(19) Europäisches Patentamt
European Patent Office
Office européen des brevets

(11) **EP 2 187 869 B1**

(12) **EUROPEAN PATENT SPECIFICATION**

(45) Date of publication and mention of the grant of the patent:
**14.10.2015 Bulletin 2015/42**

(21) Application number: **08871849.9**

(22) Date of filing: **14.08.2008**

(51) Int Cl.:
**A61K 9/127** [(2006.01)]

(86) International application number:
**PCT/US2008/073185**

(87) International publication number:
**WO 2009/097011 (06.08.2009 Gazette 2009/32)**

(54) **IMPROVED PLATINUM DRUG FORMULATIONS**

VERBESSERTE PLATIN-ARZNEIMITTELFORMULIERUNGEN

PRÉPARATIONS AMÉLIORÉES DE MÉDICAMENTS AU PLATINE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **17.08.2007 US 965179 P**

(43) Date of publication of application:
**26.05.2010 Bulletin 2010/21**

(73) Proprietor: **Celator Pharmaceuticals, Inc.**
**Princeton, NJ 08540 (US)**

(72) Inventors:
• **TARDI, Paul**
**Princeton**
**NJ 08540 (US)**
• **JOHNSTONE, Sharon**
**Princeton**
**NJ 08540 (US)**
• **MAYER, Lawrence**
**Princeton**
**NJ 08540 (US)**

(74) Representative: **Hatzmann, Martin**
**V.O.**
**Johan de Wittlaan 7**
**2517 JR Den Haag (NL)**

(56) References cited:
**EP-A2- 0 225 130       EP-A2- 0 280 492**
**WO-A2-2007/035783    US-A1- 2006 222 696**
**US-A1- 2007 148 255    US-A1- 2007 166 368**

**Description**

Technical Field

The invention relates to compositions for use in the treatment of a hyperproliferative disease, which compositions provide for improved encapsulation and delivery of platinum-based drugs. More particularly, the invention relates to platinum drug delivery systems which provide superior therapeutic efficacy and are effectively combined with additional therapeutic agents for use in the treatment of a hyperproliferative disease.

Background Art

In recent years metal-based therapeutic agents have played a major role in chemotherapy treatments. In particular, platinum-based compounds are proving to be some of the most effective anticancer drugs used in clinical practice. Platinum agents are used primarily because they form DNA adducts that block DNA and RNA synthesis and induce apoptosis. Cisplatin, carboplatin and oxaliplatin dominate the world platinum cancer market and have become critical elements in the standard practice of care for numerous solid tumors including ovarian, lung, colorectal, testicular, bladder, gastric, melanoma and head and neck cancers. Oxaliplatin has a different mechanism of action than either cisplatin or carboplatin and has proven to be especially important in treatments against cancers that have exhibited resistance against a first-line treatment with either cisplatin or carboplatin.

One of the drawbacks of platinum-based drug use is toxicity. Common side effects include kidney and nerve damage, high-end hearing loss, and prolonged nausea and vomiting. Cisplatin, in particular, has a very short half-life in the blood which results in acute nephrotoxicity due to excretion of the drug by the kidneys. Because the drug is cleared so rapidly, administration of high doses is necessary in order to achieve therapeutic activity. Increasing the therapeutic index (increasing efficacy without increasing toxicity) of these drugs is an ongoing necessity in the field and an ideal method has not yet been disclosed.

In recent years, drug delivery vehicles have been investigated for their ability to enhance the circulation lifetime of the cisplatin in order to reduce toxicity and increase efficacy. Ideally such carrier systems should remain in circulation for extended periods of time so as to accumulate at tumor sites and gradually release the drug in order for it to exert its therapeutic effects. One of the drawbacks of current platin delivery vehicles is that formulations which are designed to load sufficient amounts of drug and circulate for extended time show little therapeutic activity. Poor bioavailability of the encapsulated platinum-based drug has been proposed to be the reason for the less than optimal activity. In this respect, the drug-containing delivery vehicles are cleared from the body before drug is released from the liposomes at the disease site. Other liposome formulations release the drug rapidly and show some activity, yet not optimal. It is likely here that the released drug is cleared from the body before it has adequate time to accumulate at the disease site.

SPI-077 is a pegylated liposomal cisplatin formulation made of hydrogenated soy phosphatidylcholine (HSPC), distearyl phosphatidylethanolamine-polyethylene glycol (DSPE-PEG) and cholesterol. It has been extensively studied in clinical trials as a treatment for head and neck, ovarian and non-small cell lung cancers (see US Patent Nos. 5,945,122 and 6,126,966). The presence of the hydrophilic PEG polymer coating the inner and outer surfaces of the liposomes acts to enhance the circulation lifetime of these carriers by evading clearance by macrophages of the immune system. Although designed to load cisplatin and accumulate at the tumor site, phase II data shows that SPI-077 appears inferior to conventional cisplatin in the treatment of non-small cell lung cancer (Thatcher, et al. (1995) Ann Oncol 6:S83-S95). The authors themselves state that the minimal therapeutic activity is likely due to "insufficient release of cisplatin from the liposomes" (Meerum, et al. (2002) Cancer Chemother Pharmacol, 49:21-210); therefore, although the liposomes are able to circulate for extended periods of time, the cisplatin is not released from the liposomes and is ultimately cleared from the body along with the intact liposomes.

More recent clinical trials have looked at Lipoplatin™, a micelle-cisplatin formulation made of cisplatin-di-palmitoyl phosphatidylglycol (DPPG) complexes in at least 30% ethanol that are surrounded by a lipid bilayer composed of soy PC, DSPE-methoxy-PEG and cholesterol (see US patent 6,511,676). The presence of DPPG, a negatively charged lipid, acts to increase entrapment of cisplatin and promote conversion to its aquated form. Cisplatin has two highly reactive ligand binding sites that, when in a low chloride environment of Lipoplatin™, will complex with the negatively charged DPPG lipid. Similar to SPI-077, these liposomes are also surrounded by a PEG coating which acts to enhance the circulation lifetime of the carrier. Clinical data comparing Lipoplatin™ with free cisplatin in patients with advanced head and neck cancers sheds light on the pharmacokinetic profile of the drugs in combination with 5-FU (Jehn, et al. (2007) Anticancer Res., Jan-Feb; 27(1A):471-5). The authors state that the liposomal formulation results in "a greater body clearance and shorter half life than conventional cisplatin", and that this may confirm the clinical observation of decreased toxicity. The terminal half life for this liposomal cisplatin was less than half as long as free cisplatin and this more rapid clearance could lead to reduced therapeutic activity.

Other attempts to increase the therapeutic activity of cisplatin include encapsulation in liposomes comprised of phosphatidylethanolamine (PE) or egg phosphatidylcholine (EPC) (Hwang, et al. (2007) J. of Dermatological Science; Apr., 46(1):11-20). Although the PE liposomes encapsulated high levels of cisplatin, negligible cisplatin was loaded into the EPC liposomes. The authors report that the presence of a lone pair of electrons on the nitrogen atom of the ethanolamine head group allows the hydrated cisplatin to form a chelate and therefore remain encapsulated. However, they suggest that the absence of the lone pair of electrons in the choline head group may result in a steric barrier for cisplatin, blocking its uptake into the liposomes. The authors also report that the cisplatin-loaded EPC liposomes demonstrated weaker cytotoxicity against melanomas compared to free cisplatin.

Co-owned international PCT patent application No. WO04/087105 discloses liposome compositions capable of delivering platinum based drugs co-encapsulated with fluoropyrimidines at specific ratios. Although the liposomes were able to successfully maintain a synergistic platinum:fluoropyrimidine ratio in the blood, compositions containing a platinum agent alone were not disclosed. Liposomes described were developed solely to match the release rates of both types of drugs and optimizing release and therapeutic efficacy of the single agents was not described. In addition, liposomes disclosed in WO04/087105 were comprised of combinations of a neutral and a charged vesicle-forming lipid and "blended liposomes" as underlying the present invention which are designed specifically to enhance the therapeutic index of platinum agents were not considered or described.

EP 0280492 A2 describes the use of blended liposomes, for example, in Example 21 wherein the liposomes contain distearoyl phosphatidylcholine (DSPC) and dipalmitoyl phosphatidylcholine (DPPC), which liposomes entrap cisplatin. The liposomal membranes do not contain cholesterol (Chol), nor do they contain phosphatidylglycerol (DPPG) or distearoyl phosphatidylglycerol (DSPG).

Similarly, EP 0225130 A2 also discloses blended liposomes containing DPPC and DSPC in which cisplatin is entrapped. Again, the liposome formulations do not contain cholesterol and DPPG or DSPG.

WO 2007/035783 A2 describes compositions which comprise liposomes having the formulation DSPC/DPPC/DSPG/Chol at a molar ratio of 35:35:20:10, wherein cisplatin and a cytidine analog, such as gemcitabine, are separately loaded in liposome formulations and administered to tumor cell models.

Although improvements have been reported in the art, an ideal formulation which successfully balances platinum drug encapsulation and release, as well as reduced toxicity and increased efficacy has not been disclosed. The present inventors have identified blended liposome formulations in which the mixture of neutral vesicle-forming phosphatidylcholine lipids such as distearyl phosphatidylcholine (DSPC) and dipalmitoyl phosphatidylcholine (DPPC) results in an optimal balance of platinum-drug encapsulation and intermediate release rates that leads to a significant increase in the therapeutic index of these drugs.

As mentioned above, one of the major hurdles in platinum-drug therapies is toxicity. A further complication to this toxicity is the fact that combinations of drugs are typically used to treat metastatic disease. Therefore, the dose-limiting toxicity of additional drugs in the combination may act to lower the allowable platinum-drug concentration. For this reason, formulations capable of delivering platinum-based drugs with additional therapeutic agents need to be identified. Of particular interest is the combination of irinotecan or other camptothecin derivatives with cisplatin.

Cisplatin/irinotecan combinations have been broadly examined preclinically both *in vitro* and *in vivo.* Evidence of synergy has been observed when the two free drugs are exposed simultaneously to human lung tumor cells in culture (Takada, et al., Proc. Amer. Assoc. Cancer Res. (1992) 33: 226 and Kano, et al., Int. J. Cancer (1992) 50: 604) and increased antitumor activity obtained for the combination in two human lung cancer xenograft models was suggestive of *in vivo* synergy (Kudoh, et al., Jpn. J. Cancer Res. (1993) 84:203.) Similarly, US patent No. 6,545,010 shows synergy of these unencapsulated agents in the treatment of mice bearing colon adenocarcinomas. It has been hypothesized that these two drugs may synergize by virtue of irinotecan's inhibition of topoisomerase I activity which is required to repair DNA lesions caused by cisplatin-DNA adducts. More recently, *in vitro* studies in a non-small cell lung cancer line indicated that the synergy between irinotecan and cisplatin was dependent on the ratio of the two drugs (see co-owned U.S. patent application 2003/0147945) and that this ratio dependency was not associated with alterations of intracellular drug accumulation (Zastre, et al., Cancer Chemother. Pharmacol. 2006 (Online First)). U.S. patent application 2003/0147945 discusses administration of synergistic combinations of cisplatin and irinotecan; however, blended liposome formulations underlying the current invention designed to optimize the *in vivo* activity of cisplatin were not described.

It has been found that delivering fixed, synergistic ratios of irinotecan:cisplatin using novel blended liposomal formulations underlying the present invention could significantly improve the therapeutic index of these drugs combined.

Disclosure of the Invention

The invention relates to compositions for use in the treatment of a hyperproliferative disease, which compositions increase the therapeutic index of platinum-based therapeutic agents. These compositions allow for adequate drug loading, optimized drug release, reduced toxicity and superior efficacy. Blended lipid-based delivery vehicles are comprised of a mixture of phosphatidylcholine lipids with varying acyl chain lengths. These formulations result in a fine balance of drug encapsulation and release. As seen here, intermediate platinum-drug release allows for both enhanced therapeutic efficacy and reduced toxicity.

Thus, the present invention provides a composition for use in treating a hyperproliferative disease in a subject, which composition comprises:

> liposomes that consist of phosphatidylglycerol (DPPG) or distearoyl phosphatidylglycerol (DSPG); cholesterol (Chol); and equimolar amounts of distearoyl phosphatidylcholine (DSPC) and dipalmitoyl phosphatidylcholine (DPPC) encapsulating a platinum-based drug;
> in combination with a water-soluble camptothecin encapsulated in liposomes, wherein the liposomes encapsulating the platinum-based drug consist of DSPC:DPPC:DSPG:Chol or DSPC:DPPC:DPPG:Chol at the molar ratio 35:35:20:10.

The compositions allow the platinum-based drug and the camptothecin to be delivered to the disease site in a coordinated fashion, thereby assuring that the agents will be present at the disease site at a desired ratio. This result will be achieved whether the agents are co-encapsulated in the above-specified blended lipid-based delivery vehicles (liposomes), or are separately encapsulated in blended lipid-based delivery vehicles administered such that desired ratios are maintained at the disease site. The pharmacokinetics (PK) of the composition are controlled by the blended lipid-based delivery vehicles themselves such that coordinated delivery is achieved (provided that the PK of the delivery systems are comparable).

A method to deliver a therapeutically effective amount of a platinum-based drug/therapeutic agent combination to a desired target by administering the compositions underlying the invention is described herein.

In one aspect, the blended liposome composition for parenteral administration comprises a platinum-based drug and a water-soluble camptothecin associated with the liposomes at therapeutically effective ratios that are able to exert a desired, preferably synergistic, effect. The therapeutically effective ratio of the agents is determined by assessing the biological activity or effects of the agents on relevant cell culture or cell-free systems, as well as tumor cell homogenates from individual patient biopsies, over a range of concentrations. Any method which results in determination of a ratio of agents which maintains a desired therapeutic effect may be used. By "relevant" cells, applicants refer to at least one cell culture or cell line which is appropriate for testing the desired biological effect. As these agents are used as antineoplastic agents, "relevant" cells are those of cell lines identified by the Developmental Therapeutics Program (DTP) of the National Cancer Institute (NCI)/National Institutes of Health (NIH) as useful in their anticancer drug discovery program. Currently the DTP screen utilizes 60 different human tumor cell lines. The desired activity on at least one of such cell lines would need to be demonstrated. By "tumor cell homogenate", the applicant refers to cells generated from the mechanical or chemical disruption of patient biopsies or tumors into whole cell samples. Extraction of whole tumors or tumor biopsies can be achieved through standard medical techniques by a qualified physician and homogenization of the tissue into single, whole cells can be carried out in the laboratory using a number of methods well-known in the art. Preferred combinations are irinotecan and cisplatin or irinotecan and carboplatin.

A method to deliver a therapeutically effective amount of a platinum drug/therapeutic agent combination involves administering a platinum-based drug stably associated with a first blended delivery vehicle and an additional therapeutic agent stably associated with a second delivery vehicle. The first and second delivery vehicles may be contained in separate vials, the contents of the vials being administered to a patient simultaneously or sequentially. In one embodiment, the ratio of the platinum-based drug and the therapeutic agent administered is non-antagonistic.

As further described below, preferably, in designing an appropriate combination in accordance with the method described above, the non-antagonistic ratios are selected as those that have a combination index (CI) of $\leq 1.1$. The non-antagonistic ratio of the agents is determined by assessing the biological activity or effects of the agents on relevant cell culture or cell-free systems over a range of concentrations and, in one embodiment, applying an algorithm to determine a "combination index," (CI) where CI< 1 = synergy; CI $\sim$ 1 = additivity and CI > 1 = antagonism. As further described below, using recognized algorithms, a combination index can be calculated at each concentration level. Ratios are preferably selected where the CI represents synergy or additivity over a range of concentrations. Preferably the CI is synergistic over a wide concentration range.

The blended liposomal formulations are designed such that they stably incorporate an effective amount of a platinum drug/therapeutic agent combination and allow for the sustained release of both drugs *in vivo.*

In a further aspect, the compositions provide for the administration of effective amounts of camptothecin/cisplatin combinations using blended liposomal vehicles that are stably associated with at least one water-soluble camptothecin and cisplatin. The composition comprises at least one water-soluble camptothecin and cisplatin in a mole ratio of the water-soluble camptothecin to cisplatin which exhibits a desired, preferably synergistic biologic effect to relevant cells in culture or cell-free systems and tumor cell homogenates. Preferably, the water-soluble camptothecin is irinotecan.

Brief Description of the Drawings

FIGURE 1 is a graph showing the half-life of cisplatin versus the efficacy (% Increase in Lifespan, %ILS) of the formulation in various DSPC and/or DPPC blended liposomes.

FIGURE 2 is an outline showing the transition melting temperature and the maximum tolerated dose of cisplatin or gemcitabine in various DSPC and/or DPPC blended liposomes.

FIGURE 3 is a graph comparing the activity of a free cisplatin (open circles) versus liposomal-cisplatin (filled triangles) in DSPC/DPPC/DSPG/Cholesterol (35:35:20:10) liposomes or saline control (filled circles) given to mice bearing human HCT 116 colon xenograft tumors.

FIGURE 4A is a graph of the CI for irinotecan:cisplatin at various mole ratios as a function of the fraction of human H460 cells affected ($f_a$).

FIGURE 4B is a graph of the CI for cisplatin:edelfosine at various mole ratios as a function of the fraction of human H460 cells affected ($f_a$).

FIGURE 4C is a graph of the CI for cisplatin:daunorubicin at various mole ratios as a function of the fraction of human H460 cells affected ($f_a$).

FIGURE 4D is a graph of the CI for cisplatin:topotecan at various mole ratios as a function of the fraction of human H460 cells affected ($f_a$).

FIGURE 5 is a graph of the irinotecan (filled circles) and cisplatin (open circles) concentrations in plasma (nmoles/mL) as a function of time after intravenous administration in separate blended DSPC/DPPC/DSPG/Chol (35/35/20/10) liposomes when the drugs are administered simultaneously at an irinotecan:cisplatin mole ratio of 5:1.

FIGURE 6A is a graph comparing the activity of a free drug cocktail of irinotecan and cisplatin (filled squares), irinotecan and cisplatin co-administered in a blended liposome formulation (filled triangles) or saline control (filled circles) given to mice bearing Capan-1 human pancreatic xenograft tumors. Irinotecan and cisplatin were separately formulated in DSPC/DPPC/DSPG/Cholesterol (35/35/20/10) liposomes and were combined and co-injected at a 5:1 mole ratio. The arrows along the x-axis indicate the dosing schedule.

FIGURE 6B is a graph comparing the activity of a free drug cocktail of irinotecan and cisplatin at two different dosing schedules (filled triangles and open squares), irinotecan and cisplatin co-administered in a blended liposome formulation (open triangles) or saline control (filled circles) given to mice bearing human H460 non-small cell lung cancer solid tumors. Irinotecan and cisplatin were separately formulated in DSPC/DPPC/DSPG/Cholesterol (35/35/20/10) liposomes and were combined and co-injected at a 7:1 mole ratio. The arrows along the x-axis indicate the dosing schedule.

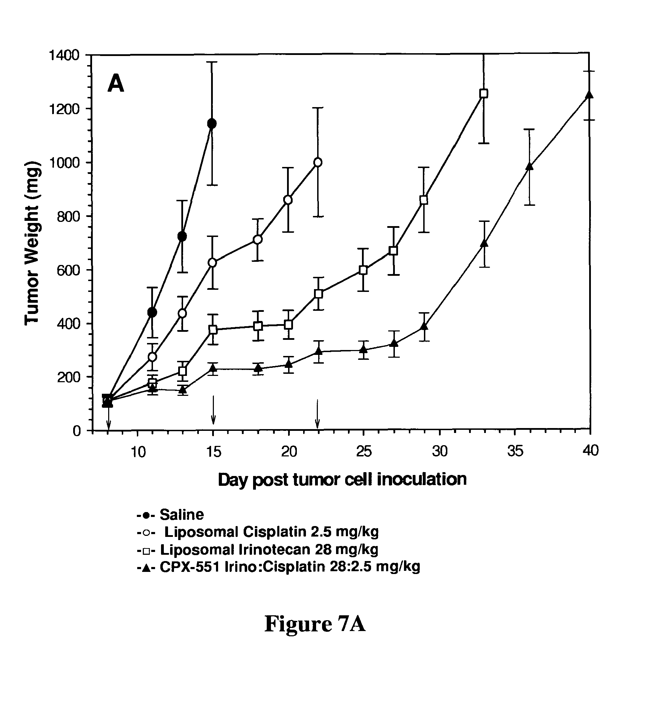

FIGURE 7A is a graph comparing the activity of irinotecan and cisplatin co-administered in a blended liposome formulation (filled triangles), liposomal irinotecan alone (open squares) or liposomal cisplatin alone (open circles) as well as a saline control (filled circles) given to mice bearing human H460 non-small cell lung cancer solid tumors. Irinotecan and cisplatin were separately formulated in blended DSPC/DPPC/DSPG/Cholesterol (35/35/20/10) liposomes. When co-injected, the drugs were combined at a 5:1 irinotecan:cisplatin mole ratio. The arrows along the x-axis indicate the dosing schedule.

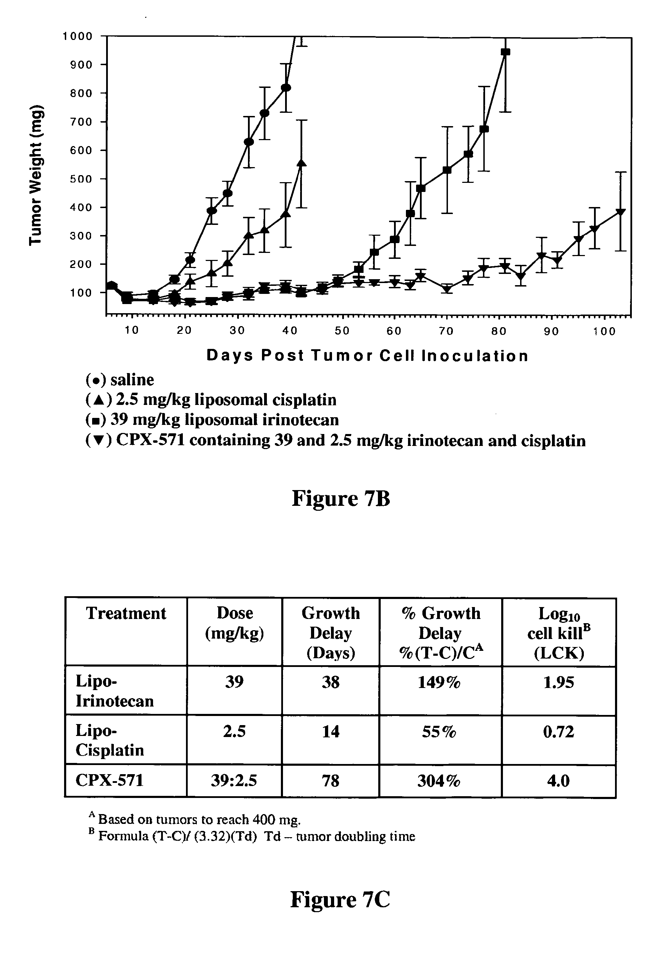

FIGURE 7B is a graph comparing the activity of irinotecan and cisplatin co-administered in a blended liposome formulation (filled triangles), liposomal irinotecan alone (open squares) or liposomal cisplatin alone (open circles) as well as a saline control (filled circles) given to mice bearing human H69 small cell lung cancer (SCLC) solid tumors. Irinotecan and cisplatin were separately formulated in blended DSPC/DPPC/DSPG/Cholesterol (35/35/20/10) liposomes. When co-injected, the drugs were combined at a 7:1 irinotecan:cisplatin mole ratio. The arrows along the x-axis indicate the dosing schedule.

FIGURE 7C is a table outlining the percent growth delay and log cell kill (LCK) values for liposomal irinotecan and liposomal cisplatin alone and co-administered at a 7:1 ratio (CPX-571).

FIGURE 8 is a graph showing the antitumor efficacy (as log cell kill) of irinotecan and cisplatin co-administered in blended liposomes at both a synergistic 5:1 ratio and an antagonistic 1:2 ratio to mice bearing H1299 NSCLC human xenograft tumors.

Modes of Carrying Out the Invention

As noted herein above, the compositions underlying the invention comprise blended lipid-based delivery vehicles (liposomes) stably associated with a platinum-based drug and one additional therapeutic agent (i.e., a camptothecin), wherein the platinum-based drug and additional therapeutic agent are present at platinum drug/therapeutic agent mole ratios that exhibit a desired cytotoxic, cytostatic or biologic effect to relevant cells or tumor cell homogenates. Preferably the desired cytotoxic, cytostatic or biologic effect is non-antagonistic.

The blended lipid-based delivery vehicles are liposomes consisting of a mixture of DSPC, DPPC, DSPG or DPPG, and cholesterol at the molar ratio 35:35:20:10.

In one embodiment, blended liposomal compositions for use according to the invention will include the above-described liposomes stably associated with either cisplatin or carboplatin and irinotecan, preferably, at a cisplatin (or carboplatin):irinotecan mole ratio which is non-antagonistic. The mole ratio of irinotecan to cisplatin may be greater than 4:1 or about 1:4 or less.

The above described blended lipid-based delivery vehicle compositions may comprise a third or fourth agent. Any therapeutic, diagnostic or cosmetic agent may be included.

"Encapsulation," includes covalent or non-covalent association of an agent with the blended lipid-based delivery vehicle. For example, this can be by interaction of the agent with the outer layer or layers of the liposome or entrapment of an agent within the liposome, equilibrium being achieved between different portions of the liposome. Thus encapsulation of an agent can be by association of the agent by interaction with the bilayer of the blended liposomes through covalent or non-covalent interaction with the lipid components or entrapment in the aqueous interior of the liposome, or in equilibrium between the internal aqueous phase and the bilayer. "Loading" refers to the act of encapsulating one or more agents into a delivery vehicle.

When combinations of drugs are used, it is important that the therapeutic agents be "stably associated" with the delivery vehicles. By "stably associated" is meant that the compositions are such that when the agents are supplied in a mole ratio that is non-antagonistic, a non-antagonistic ratio is maintained in the blood of a subject for at least 1 hour after administration to the subject. In some embodiments, a non-antagonistic ratio is maintained for 4 or more hours, even for 24 hours.

Thus, in the compositions underlying the present invention, the platinum-based drug and additional pharmaceutical agent (camptothecin) may be administered together in a composition in a non-antagonistic ratio, in which case stable association with the delivery vehicles is needed. In one embodiment, the platinum-based drug and the additional therapeutic agent (camptothecin) are encapsulated together in the above-specified blended liposomes. In another embodiment, the platinum-based drug is stably associated with the above-specified blended liposomes and the other member of the combination (camptothecin) is stably associated with a delivery vehicle (liposomes) having pharmacokinetic properties coordinated with the platinum-based drug contained in the blended liposomes. The other delivery vehicle is not limited to the above-specified blended liposomes. However, when co-administered, each agent contained in its own delivery vehicles, the combination must maintain a non-antagonistic ratio in the blood of the subject for at least an hour or more.

The above-specified blended lipid-based delivery vehicles may be used not only in parenteral administration but also in topical, nasal, subcutaneous, intraperitoneal, intramuscular, aerosol or oral delivery or by the application of the delivery vehicle onto or into a natural or synthetic implantable device at or near the target site for therapeutic purposes or medical imaging and the like. Preferably, the above-specified blended lipid-based delivery vehicles of the invention are used in parenteral administration, most preferably, intravenous administration.

The preferred embodiments herein described are not intended to be exhaustive or to limit the scope of the invention to the precise forms disclosed. They are chosen and described to best explain the principles of the invention and its application and practical use to allow others skilled in the art to comprehend its teachings.

Preparation of Blended Lipid-Based Delivery Vehicles

"Blended" lipid carriers for use in the compositions underlying this invention are liposomes that consist of phosphatidylglycerol (DPPG) or distearoyl phosphatidylglycerol (DSPG), cholesterol, distearoyl phosphatidylcholine (DSPC) and dipalmitoyl phosphatidylcholine (DPPC) wherein the molar ratio of DSPC:DPPC:DSPG:Chol or DSPC:DPPC:DPPG:Chol is 35:35:20:10. Accordingly, the "blended liposomes", "blended lipid-based delivery vehicles" and "blended lipid carriers" are liposomes which include a mixture of phosphatidylcholine lipids of varying acyl chain lengths: a mixture of equimolar amounts of the phosphatidylcholine lipids, DSPC and DPPC. Blended liposomes of the invention can be prepared as described in Liposomes: Rational Design (A.S. Janoff, ed., Marcel Dekker, Inc., New York, NY), or by additional techniques known to those knowledgeable in the art. Suitable liposomes for use in the compositions underlying this invention include large unilamellar vesicles (LUVs), multilamellar vesicles (MLVs), small unilamellar vesicles (SUVs) and interdigitating fusion liposomes.

As noted herein above, blended liposomes of the compositions underlying the invention also contain the sterol, cholesterol.

The incorporation of the negatively charged lipid, phosphatidylglycerol (DPPG), to liposome formulations increases the circulation longevity of the carrier. The compositions underlying this invention may make use of low-cholesterol blended liposomes containing DPPG to prevent aggregation, thereby increasing the blood residence time of the carrier.

In one embodiment, blended liposome compositions for use in accordance with this invention are preferably used to treat cancer, including drug resistant cancers such as cisplatin-resistant cancers. Delivery of encapsulated drugs to a tumor site is achieved by administration of the above-specified blended liposomes. Preferably, the blended liposomes have a diameter of less than 300 nm. Most preferably, the blended liposomes have a diameter of less than 200 nm. Tumor vasculature is generally leakier than normal vasculature due to fenestrations or gaps in the endothelia. This allows the delivery vehicles of 200 nm or less in diameter to penetrate the discontinuous endothelial cell layer and underlying basement membrane surrounding the vessels supplying blood to a tumor. Selective accumulation of the delivery vehicles into tumor sites following extravasation leads to enhanced anticancer drug delivery and therapeutic effectiveness.

Various methods may be utilized to encapsulate therapeutic agents in blended liposomes.

Encapsulation of the desired combination can be achieved either through encapsulation in separate delivery vehicles or within the same blended delivery vehicle. Where encapsulation into separate liposomes is desired, the lipid composition of each liposome may be different to allow for coordinated pharmacokinetics so long as a platinum agent is encapsulated in a blended liposome as specified herein above. By altering the vehicle composition, release rates of encapsulated drugs can be matched to allow desired ratios of the drugs to be delivered to the tumor site. When encapsulated in separate liposomes, ratios of drugs that have been determined to provide non-antagonistic therapeutic activity, can be generated by combining the appropriate amounts of each liposome-encapsulated drug prior to administration. Alternatively, two or more agents may be encapsulated within the same blended liposome.

The therapeutic agents may be loaded into the blended liposomes using both passive and active loading methods. Platinum-based drugs such as cisplatin are typically passively loaded. Passive methods of encapsulating drugs in liposomes most often involve encapsulating the agent during the preparation of the liposomes. This includes a passive entrapment method described by Bangham, et al. (J. Mol. Biol. (1965) 12:238). This technique results in the formation of multilamellar vesicles (MLVs) that can be converted to large unilamellar vesicles (LUVs) or small unilamellar vesicles (SUVs) upon extrusion. Additional suitable methods of passive encapsulation include an ether injection technique described by Deamer and Bangham (Biochim. Biophys. Acta (1976) 443:629) and the Reverse Phase Evaporation technique as described by Szoka and Papahadjopoulos (P.N.A.S. (1978) 75:4194). Agents such as cisplatin can also be passively loaded into preformed liposomes as described in the Examples.

Active methods of encapsulation include the pH gradient loading technique described in U.S. patent Nos. 5,616,341, 5,736,155 and 5,785,987 and active metal-loading. Camptothecin agents are typically actively loaded. A preferred method of pH gradient loading is the citrate-base loading method utilizing citrate as the internal buffer at a pH of 4.0 and a neutral exterior buffer. Other methods employed to establish and maintain a pH gradient across a liposome involve the use of an ionophore that can insert into the liposome membrane and transport ions across membranes in exchange for protons (see U.S. patent No. 5,837,282). A recent technique utilizing transition metals to drive the uptake of drugs into liposomes via complexation in the absence of an ionophore may also be used (see co-owned U.S. patent No. 7,238,367). This technique relies on the formation of a drug-metal complex rather than the establishment of a pH gradient to drive uptake of drug. An additional active loading technique described in co-owned international PCT application WO07/076117 makes use of a secondary or tertiary amine aqueous solution in the intraliposomal space to drive uptake of drugs containing protonatable amine groups.

Passive and active methods of entrapment may also be coupled in order to prepare a liposome formulation containing more than one encapsulated agent.

As noted above, only the platinum-based therapeutic agent need be encapsulated with the above-specified blended liposomes; the other agent (camptothecin) may be supplied in an alternate delivery vehicle with pharmacokinetics coordinated with those of the blended liposomal portion.

Platinum Agents

In recent years metal-based therapeutic agents have played a relevant role in chemotherapy treatments. In particular, platinum-based compounds, are proving to be some of the most effective anticancer drugs used in clinical practice. Platinum agents are used primarily because they form DNA adducts that block DNA and RNA synthesis and apoptosis. The nature of the platinum/DNA adducts has been widely investigated by hydrolysis of DNA into nucleotides. Studies have shown that the adduct is typically a cross link involving the N-7 of DNA purine (adenine (A) and guanine (G)) bases. The preferential complex for platinum compounds such as cisplatin, is an intrastrand crosslink at the dinucleotides GG (62% occurrence) and AG (22% occurrence). In fact, intrastrand cross linking has been shown to correlate with the clinical response to cisplatin therapy. To possess this type of antitumor activity, a platinum agent must have two relatively labile leaving groups to react with the DNA bases. Typically, platinum agents have a central platinum atom bonded to four ligands, two of which are reactive. In the case of cisplatin, the platinum atom is linked to two amino groups and two chloride (leaving) groups.

"Platinum agents" and "platinum-based drugs," therefore refers to therapeutic drugs that contain a reactive platinum atom, including derivatized and underivatized forms of cisplatin and its related compounds which have the essential features of containing a reactive platinum atom.

Approximately 3,000 platinum analogs have been synthesized over the past 30 years; however, only 6 are presently in clinical use, including cisplatin, carboplatin and oxaliplatin. Cisplatin, carboplatin and oxaliplatin dominate the world platinum/cancer market and have become critical elements in the standard practice of care for numerous solid tumors including ovarian, lung, colorectal, testicular, bladder, gastric, melanoma and head and neck cancers. Oxaliplatin has a different mechanism of action than either cisplatin or carboplatin which has proven to be especially important in cisplatin-resistant models and cell lines expressing resistance genes.

Cisplatin (*cis*-diamminedichoroplatinum (II)) has a well-established activity spectrum, mainly in the treatment of lung, ovarian, and germ cell tumors. Its binding to DNA has been shown to cause the DNA duplex to bend and unwind. This coordination to the DNA leads to not only inhibition of DNA replication and transcription, but also to increased apoptosis. Interestingly, the cytotoxic activity of cisplatin is dependent upon its stereochemistry, as the trans isomer (which binds DNA differently) exhibits reduced activity compared to the cis isomer.

Developments of structurally related cisplatin analogs (of the cis isomer), such as carboplatin and oxaliplatin, have focused on platinum-based complexes which may act through different mechanisms of action than cisplatin. Ideally these agents would demonstrate reduced toxicity and/or enhanced activity against cisplatin-resistant cancers.

Carboplatin (cis-diammine-1,1-cyclobutanedicarboxylateplatinum) is one of the best-characterized cisplatin analogs. It was approved by the United States Food and Drug Administration (FDA) in 1989 for treatment of human ovarian cancers. It is known to form an identical type of DNA adduct and exhibit the same therapeutic effects as cisplatin. However, carboplatin requires lower doses to achieve these same affects and it displays significantly less toxicity to the peripheral nervous system and kidneys. It is believed that this is likely due to the presence of a bidentate dicarboxylate ligand in carboplatin which slows down its degradation into potentially damaging derivatives.

Oxaliplatin (trans-1-diaminocyclohexane oxalatoplatinum) is a relatively new platinum salt that belongs to the DACH (diaminocyclohexane) platinum family, and is the only such cisplatin analog that has entered clinical development and achieved approval for marketing. It demonstrates good clinical tolerance with the absence of renal or auditory toxicity. The exact mechanism of action of oxaliplatin is unclear. It is known to form reactive platinum complexes which are believed to inhibit DNA synthesis by forming interstrand and intrastrand cross-linking of DNA molecules; however, it binds in a different location on DNA than cisplatin or carboplatin. It is also a larger and bulkier compound than either cisplatin or carboplatin, which makes it harder to separate from the DNA once bound. Another difference between oxaliplatin and the other platinum compounds is its spectrum of cytotoxicity, or ability to induce cell death. In particular, it has shown activity against six colorectal cell lines that both cisplatin and carboplatin have limited cytotoxicity, demonstrating the selective nature of these platinum-based compounds.

In embodiments of the invention, the "platinum agent" is selected based on its activity against a particular cell type or tumor. In preferred embodiments, the platinum agent is cisplatin, carboplatin or oxaliplatin. Most preferably, the platinum agent is cisplatin.

The effects of combining camptothecins and platinum agents in blended liposomes will act to inhibit DNA synthesis through multiple pathways and likely induce apoptosis. This is critical for the treatment of hyperproliferative diseases such as cancer, and importantly, may prove superior for treating drug resistant cancers such as cisplatin-resistant tumors.

Therapeutic Agents for Combinations with Platinum-based Drugs

Various combinations of therapeutic agents and platinum-based drugs, having been found to satisfy the criteria for non-antagonistic effects set forth above, are then provided in the form of formulations of blended drug delivery vehicles. A "therapeutic agent" is a compound that alone, or in combination with other compounds, has a desirable effect on a subject affected by an unwanted condition or disease.

Certain therapeutic agents are favored for use in combination with platinum-based drugs when the target disease or condition is cancer. Examples are:

"Topoisomerase inhibitors", such as camptothecins, which interfere with topoisomerase I or II activity, enzymes necessary for DNA replication and transcription;

"Antimetabolites," such as Gemcitabine or 5-FU, which closely resemble an essential metabolite and therefore interfere with physiological reactions involving it;

"Signal transduction inhibitors" which interfere with or prevents signals that cause cancer cells to grow or divide;

"Cytotoxic agents";

"Cell cycle inhibitors" or "cell cycle control inhibitors" which interfere with the progress of a cell through its normal cell cycle, the life span of a cell, from the mitosis that gives it origin to the events following mitosis that divides it into daughter cells;

"Checkpoint inhibitors" which interfere with the normal function of cell cycle checkpoints, e.g., the S/G2 checkpoint, G2/M checkpoint and G1/S checkpoint;

"Receptor tyrosine kinase inhibitors" which interfere with the activity of growth factor receptors that possess tyrosine kinase activity;

"Apoptosis inducing agents" which promote programmed cell death;

"Telomerase inhibitors" which interfere with the activity of a telomerase, an enzyme that extends telomere length and extends the lifetime of the cell and its replicative capacity;

"Cyclin-dependent kinase inhibitors" which interfere with cyclin-dependent kinases that control the major steps between different phases of the cell cycle through phosphorylation of cell proteins such as histones, cytoskeletal proteins, transcription factors, tumor suppresser genes and the like;

"DNA damaging agents";

"DNA repair inhibitors";

"Anti-angiogenic agents" which interfere with the generation of new blood vessels or growth of existing blood vessels that occurs during tumor growth; and

"Mitochondrial poisons" which directly or indirectly disrupt mitochondrial respiratory chain function.

In one embodiment, combinations for treatment of tumors are cisplatin (or carboplatin) and irinotecan, and cisplatin (or carboplatin) and topotecan.

Determining Non-antagonistic Platinum Drug/Camptothecin Ratios *In Vitro*

In one aspect of the compositions underlying the invention, platinum agents and camptothecins will be encapsulated in the blended lipid-based delivery vehicles at synergistic or additive (*i.e.,* non-antagonistic) ratios. Determination of ratios of agents that display synergistic or additive combination effects may be carried out using various algorithms, based on the types of experimental data described below. These methods include isobologram methods (Loewe, et al., Arzneim-Forsch (1953) 3:285-290; Steel, et al., Int. J. Radiol. Oncol. Biol. Phys. (1979) 5:27-55), the fractional product method (Webb, Enzyme and Metabolic Inhibitors (1963) Vol. 1, pp. 1-5. New York: Academic Press), the Monte Carlo simulation method, CombiTool, ComboStat and the Chou-Talalay median-effect method based on an equation described in Chou, J. Theor. Biol. (1976) 39:253-76; and Chou, Mol. Pharmacol. (1974) 10:235-247). Alternatives include surviving fraction (Zoli, et al., Int. J. Cancer (1999) 80:413-416), percentage response to granulocyte/macrophage-colony forming unit compared with controls (Pannacciulli, et al., Anticancer Res. (1999) 19:409-412) and others (Berenbaum, Pharmacol. Rev. (1989) 41:93-141; Greco, et al., Pharmacol Rev. (1995) 47:331-385).

The Chou-Talalay median-effect method is preferred. The analysis utilizes an equation wherein the dose that causes a particular effect is given by:

$$D = D_m[f_a/(1-f_a)]^{1/m}$$

in which D is the dose of the drug used, $f_a$ is the fraction of cells affected by that dose, $D_m$ is the dose for median effect signifying the potency and m is a coefficient representing the shape of the dose-effect curve (m is 1 for first order reactions).

This equation can be further manipulated to calculate a combination index (CI) on the basis of the multiple drug effect equation as described by Chou and Talalay, Adv. Enzyme Reg. (1984) 22:27-55; and by Chou, et al., in: Synergism and Antagonism in Chemotherapy, Chou and Rideout, eds., Academic Press: New York (1991) 223-244. A computer program (CalcuSyn) for this calculation is found in Chou and Chou ("Dose-effect analysis with microcomputers: quantitation of ED50, LD50, synergism, antagonism, low-dose risk, receptor ligand binding and enzyme kinetics": CalcuSyn Manual and Software; Cambridge: Biosoft 1987).

The combination index equation is based on the multiple drug-effect equation of Chou-Talalay derived from enzyme kinetic models. An equation determines only the additive effect, rather than synergism and antagonism. However, according to the CalcuSyn program, synergism is defined as a more than expected additive effect, and antagonism as a less than expected additive effect. Chou and Talalay in 1983 proposed the designation of CI=1 as the additive effect, thus from the multiple drug effect equation of two drugs, we obtain:

$$CI = (D)_1/(D_x)_1 + (D)_2/(D_x)_2 \quad [Eq. 1]$$

for mutually exclusive drugs that have the same or similar modes of action, and it is further proposed that

$$CI = (D)_1/(D_x)_1 + (D)_2/(D_x)_2 + (D_1)(D_2)/(D_x)_1(D_x)_2 \quad [Eq.\ 2]$$

for mutually non-exclusive drugs that have totally independent modes of action. CI <1, = 1, and >1 indicates synergism, additive effect, and antagonism, respectively. Equation 1 or equation 2 dictates that drug 1, $(D)_1$, and drug 2, $(D)_2$, (in the numerators) in combination inhibit x % in the actual experiment. Thus, the experimentally observed x % inhibition may not be a round number but most frequently has a decimal fraction. $(D_x)_1$ and $(D_x)_2$ (in the denominators) of equations 1 and 2 are the doses of drug 1 and drug 2 alone, respectively, inhibiting x %.

For simplicity, mutual exclusivity is usually assumed when more than two drugs are involved in combinations (CalcuSyn Manual and Software; Cambridge: Biosoft 1987).

A two-drug combination may be further used as a single pharmaceutical unit to determine synergistic or additive interactions with a third agent. In addition, a three-agent combination may be used as a unit to determine non-antagonistic interactions with a fourth agent, and so on.

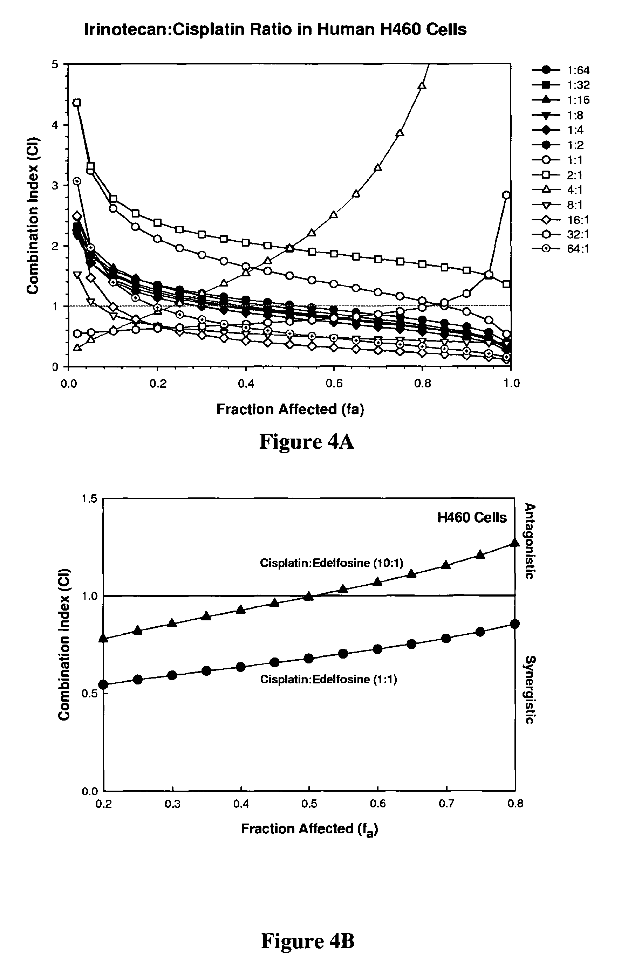

The underlying experimental data are generally determined *in vitro* using cells in culture or in cell-free systems. Preferably, the combination index (CI) is plotted as a function of the fraction of cells affected ($f_a$) as shown in Figures 4A-4D which, as explained above, is a surrogate parameter for concentration range. Preferred combinations of agents are those that display synergy or additivity over a substantial range of $f_a$ values. Combinations of agents are selected that display synergy over at least 5% of the concentration range wherein greater than 1% of the cells are affected, *i.e.,* an $f_a$ range greater than 0.01. Preferably, a larger portion of overall concentration exhibits a favorable CI; for example, 5% of an $f_a$ range of 0.2-0.8. More preferably 10% of this range exhibits a favorable CI. Even more preferably, 20% of the $f_a$ range, preferably over 50 % and most preferably over at least 70% of the $f_a$ range of 0.2 to 0.8 are utilized in the compositions. Combinations that display synergy over a substantial range of $f_a$ values may be re-evaluated at a variety of agent ratios to define the optimal ratio to enhance the strength of the non-antagonistic interaction and increase the $f_a$ range over which synergy is observed.

As set forth above, the *in vitro* studies on cell cultures will be conducted with "relevant" cells. The choice of cells will depend on the intended therapeutic use of the agent. *In vitro* studies on individual patient biopsies or whole tumors will be conducted with "tumor cell homogenates," generated from the mechanical or chemical disruption of the tumor sample(s) into single, whole cells.

In one preferred embodiment, the given effect refers to cell death or cell stasis after application of a cytotoxic agent to a "relevant" cell culture (see Example 2). Cell death or viability may be measured using a number of methods known in the art, for example, the "MTT" assay (Mosmann, J. Immunol. Methods (1983) 65(1-2):55-63).

Administering Compositions underlying the Invention *In Vivo*

As mentioned above, the blended delivery vehicle compositions underlying the present invention may be administered to warm-blooded animals, including humans as well as to domestic avian species. For treatment of human ailments, a qualified physician will determine how the compositions should be utilized with respect to dose, schedule and route of administration using established protocols. Such applications may also utilize dose escalation should agents encapsulated in delivery vehicle compositions underlying the present invention exhibit reduced toxicity to healthy tissues of the subject.

Preferably, the pharmaceutical compositions for use according to the present invention are administered parenterally, i.e., intraarterially, intravenously, intraperitoneally, subcutaneously, or intramuscularly. More preferably, the pharmaceutical compositions are administered intravenously or intraperitoneally by a bolus injection. For example, see Rahman, et al., U.S. patent No. 3,993,754; Sears, U.S. patent No. 4,145,410; Papahadjopoulos, et al., U.S. patent No. 4,235,871; Schneider, U.S. patent No. 4,224,179; Lenk, et al., U.S. patent No. 4,522,803; and Fountain, et al., U.S. patent No. 4,588,578.

In other methods, the pharmaceutical preparations underlying the present invention can be contacted with the target tissue by direct application of the preparation to the tissue. The application may be made by topical, "open" or "closed" procedures. By "topical", is meant the direct application of the multi-drug preparation to a tissue exposed to the environment, such as the skin, oropharynx, external auditory canal, and the like. "Open" procedures are those procedures that include incising the skin of a patient and directly visualizing the underlying tissue to which the pharmaceutical preparations are applied. This is generally accomplished by a surgical procedure, such as a thoracotomy to access the lungs, abdominal laparotomy to access abdominal viscera, or other direct surgical approach to the target tissue. "Closed" procedures are invasive procedures in which the internal target tissues are not directly visualized, but accessed via inserting instruments through small wounds in the skin. For example, the preparations may be administered to the peritoneum by needle lavage. Alternatively, the preparations may be administered through endoscopic devices.

Pharmaceutical compositions comprising blended delivery vehicles underlying the invention are prepared according to standard techniques and may comprise water, buffered water, 0.9% saline, 0.3% glycine, 5% dextrose and the like, including glycoproteins for enhanced stability, such as albumin, lipoprotein, globulin, and the like. These compositions may be sterilized by conventional, well-known sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, and the like. Additionally, the delivery vehicle suspension may include lipid-protective agents which protect lipids against free-radical and lipid-peroxidative damages on storage. Lipophilic free-radical quenchers, such as alpha-tocopherol and water-soluble iron-specific chelators, such as ferrioxamine, are suitable.

The concentration of blended delivery vehicles in the pharmaceutical formulations can vary widely, such as from less than about 0.05%, usually at or at least about 2-5% to as much as 10 to 30% by weight and will be selected primarily by fluid volumes, viscosities, and the like, in accordance with the particular mode of administration selected. For example, the concentration may be increased to lower the fluid load associated with treatment. Alternatively, blended delivery vehicles composed of irritating lipids may be diluted to low concentrations to lessen inflammation at the site of administration. For diagnosis, the amount of delivery vehicles administered will depend upon the particular label used, the disease state being diagnosed and the judgment of the clinician.

Preferably, the pharmaceutical compositions for use according to the present invention are administered intravenously. Dosage for the blended delivery vehicle formulations will depend on the ratio of drug to lipid and the administrating physician's opinion based on age, weight, and condition of the patient.

In addition to pharmaceutical compositions, suitable formulations for veterinary use may be prepared and administered in a manner suitable to the subject. Preferred veterinary subjects include mammalian species, for example, non-human primates, dogs, cats, cattle, horses, sheep, and domesticated fowl. Subjects may also include laboratory animals, for example, in particular, rats, rabbits, mice, and guinea pigs.

Kits

The therapeutic agents in the compositions underlying the present invention containing the 2 or more active agents may be formulated separately in individual compositions wherein each therapeutic agent is stably associated with appropriate delivery vehicles provided that at least the platinum-based agent is encapsulated in the above-specified blended liposomes. These compositions can be administered separately to subjects as long as the pharmacokinetics of the delivery vehicles are coordinated so that the ratio of therapeutic agents administered is maintained for at least one hour in the blood after *in vivo* administration. Thus, it is useful to construct kits which include, in separate containers, a first composition comprising blended liposome delivery vehicles stably associated with a platinum-based drug and, in a second container, a second composition comprising delivery vehicles stably associated with at least a second therapeutic agent, namely camptothecin. The delivery vehicles in at least one composition must be the above-specified blended liposomes. The containers can then be packaged into the kit.

The kit will also include instructions as to the mode of administration of the compositions to a subject, at least including a description of the ratio of amounts of each composition to be administered. Alternatively, or in addition, the kit is constructed so that the amounts of compositions in each container is pre-measured so that the contents of one container in combination with the contents of the other represent the correct ratio. Alternatively, or in addition, the containers may be marked with a measuring scale permitting dispensation of appropriate amounts according to the scales visible. The containers may themselves be useable in administration; for example, the kit might contain the appropriate amounts of each composition in separate syringes. Formulations which comprise the pre-formulated correct ratio of therapeutic agents may also be packaged in this way so that the formulation is administered directly from a syringe prepackaged in the kit.

The following examples are offered to illustrate but not to limit the invention.

EXAMPLES

*Methods for preparation of blended liposomes*

Unless otherwise specified, lipids were dissolved in chloroform solution, mixed together at the indicated amounts, subsequently dried under a stream of nitrogen gas and placed in a vacuum pump to remove solvent. Trace levels of radioactive lipid $^{14}$C-CHE may be added to quantify lipid in appropriate experiments. The resulting lipid film was placed under high vacuum for a minimum of 2 hours. The lipid film was hydrated in the solution indicated to form multilamellar vesicles (MLVs). The resulting preparation was extruded 10 times through stacked polycarbonate filters with an extrusion apparatus (Northern Lipids Inc., Vancouver, B.C., Canada) to achieve a mean liposome size between 80 and 150 nm.

All constituent lipids of liposomes are reported in mole %. The mean diameter and size distribution of each liposome formulation was analyzed by quasi-elastic light scattering NICOMP model 380 submicron particle sizer (Pacific Scientific, Santa Barbara, CA, USA) and was typically 100 ± 20 nm.

*Methods for puantification of drug loading*

At various time points after initiation of drug loading, aliquots were removed and passed through a Sephadex G-50 spin column to separate free from encapsulated drug. To a specified volume of eluant containing encapsulated drug, Triton® X-100 or N-octyl beta-D-glucopyranoside (OGP) was added to solubilize the liposomes. Following addition of detergent, the mixture was heated to the cloud point of the detergent and allowed to cool to room temperature before measurement of the absorbance or fluorescence. Drug concentrations were calculated by comparison to a standard curve. Lipid levels were measured by liquid scintillation counting.

Example 1 (Not within scope of present invention)

Intermediate Cisplatin Release from Blended Liposomes Maximizes the Therapeutic Index

In order to determine the role of liposome composition on cisplatin activity *in vivo,* the drug half-life (time taken for the plasma concentration of a drug to reach half of its original concentration) and *in vivo* efficacy of liposomes with varying DSPC and/or DPPC content containing encapsulated cisplatin were studied.

Lipid films were prepared by dissolving different combinations of DSPC, DPPC, DSPG, DPPG and cholesterol (Chol) to generate the following liposomes: DSPC/DSPG/Chol (70/20/10 molar ratio), DSPC/DPPC/DSPG/Chol (60/10/20/10, 50/20/20/10, and 35/35/20/10 molar ratios), DSPC/DPPC/DPPG/Chol (35/35/20/10 molar ratio) and DPPC/DPPG/Chol (70/20/10 molar ratio).

Cisplatin was loaded into the liposomes above containing 150 mM NaCl by incubating the drug with the liposomes for 60 minutes at 60°C in the presence of 7.5% ethanol at a cisplatin concentration of 8 mg/ml. The reaction was cooled to precipitate unencapsulated cisplatin or diluted with hot saline. The liposomes were then buffer exchanged into saline to remove unencapsulated cisplatin and subsequently exchanged into phosphate-buffered sucrose (pH 7). Analysis of cisplatin percent drug encapsulation was determined by atomic absorption spectrometry.

The cisplatin half-life for each liposome formulation was determined by pharmacokinetic (PK) analysis in non-tumor bearing BDF-1 mice. Mice were intravenously administered 3 mg/kg encapsulated-cisplatin (375 mg/kg lipid) in the lateral tail vein. At the indicated time points (3 mice per time point) blood was collected by cardiac puncture and placed into EDTA coated microtainers. The samples were centrifuged and plasma was carefully transferred to another tube. Plasma cisplatin levels were determined by atomic absorption spectrometry and lipid levels were determined by liquid scintillation counting. For quantitation by atomic absorption spectrometry, samples were diluted in 0.1 % nitric acid to fall within the linear range of a standard curve.

The *in vivo* activity of each cisplatin liposome formulation, reported as percent (%) Increase in Life Span (ILS) was measured in female BDF-1 mice that have been injected intraperitoneally (i.p.) with P388 human leukemia cells.

Briefly, in order to perform tumor studies on mice, animals are inoculated with tumor cells which are then allowed to grow to sufficient size. Using a 28g needle, mice are inoculated i.p. with 1-2 x $10^6$ tumor cells on day 0 (one inoculum/mouse) in a volume of 50 μL. Mice were organized into appropriate treatment groups and consist of control and treatment groups such as for example, saline control, liposome control, positive control and various dilutions of test articles.

Mice were injected intravenously with the required volume of sample to administer the prescribed dose (10 μL/g as indicated) to the animals based on individual mouse weights. All animals are observed at least once a day, more if deemed necessary, during the pre-treatment and treatment periods for mortality and morbidity. In particular, signs of ill health are based on body weight loss, change in appetite, rough coat, lack of grooming, behavioral changes such as altered gait, lethargy and gross manifestations of stress. Should signs of severe toxicity or tumor-related illness be seen, the animals are euthanized ($CO_2$ asphyxiation) and a necropsy is performed to assess other signs of toxicity. Moribund animals must be terminated for humane reasons and the decision to terminate will be at the discretion of the Animal Care Technician and the Study Director/Manager. Any and all of these findings will be recorded as raw data and the time of death will be logged as the following day.

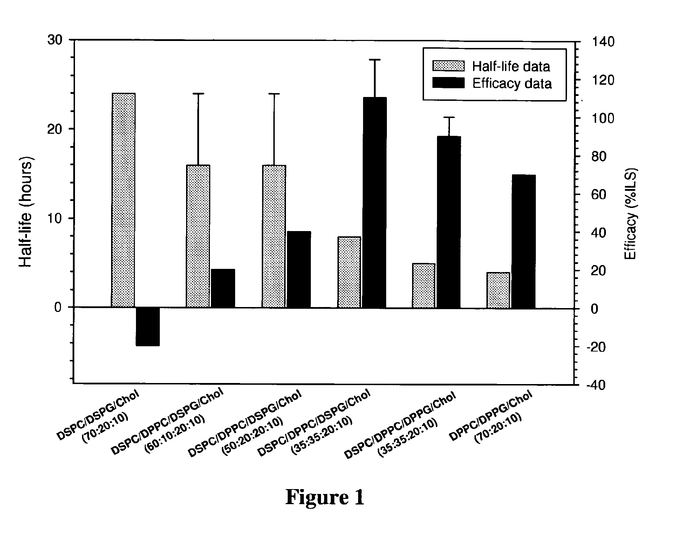

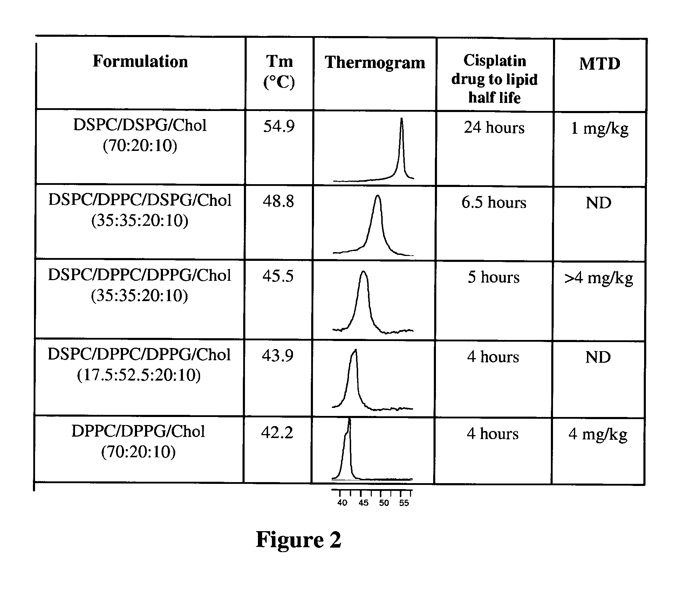

Figure 1 shows the half-life (hours) of cisplatin release versus efficacy (reported as percent ILS). As seen in the bell-shaped curve for efficacy, there is a peak formulation for efficacy which corresponds to an intermediate half-life for cisplatin release. As seen here, if the cisplatin is released too quickly or too slowly there is reduced *in vivo* efficacy. For example, the DSPC/DSPG/Chol (70/20/10) formulation demonstrated the longest half-life yet resulted in a negative % ILS. Similar results would also be expected for liposomes with high DSPC:DPPC ratios or containing very small amounts of DPPC such as DSPC/DPPC/DSPG/Chol (65/5/20/10 with a 13:1 DSPC:DPPC ratio). As DPPC was added in increasing amounts (thus decreasing the DSPC:DPPC ratio), the half-life decreased with a concomitant increase in life span until the DSPC:DPPC ratio reached 1:1. At this point, more DPPC continued to lower the half-life yet led to a slight decrease in efficacy. The table in Figure 2 shows that the maximum tolerated dose (MTD) of blended DSPC/DPPC liposomes is higher than that observed for either non-blended liposome formulations and that the most tolerated formulation comprises the DSPC/DPPC/DSPG/Cholesterol (35/35/20/10) liposomes with a 1:1 DSPC/DPPC ratio. The results displayed here indicate that a formulation with DSPC/DPPC/DSPG/Cholesterol (35/35/20/10) may provide the optimal balance of cisplatin release and efficacy *in vivo.*

Example 2 (Not within scope of present invention)

Liposomal Cisplatin in Blended Liposomes Demonstrates Increased Efficacy Compared to Free Cisplatin

The *in vivo* activity of liposomal-cisplatin delivered in DSPC/DPPC/DSPG/Cholesterol liposomes (35:35:20:10 mol %) was directly compared to the activity of an equal dose of free cisplatin. Liposomal-cisplatin was prepared as described in Example 1.

*The in vivo* activity of each cisplatin formulation, reported as decreasing tumor burden, was measured using an HCT 116 human colon xenograft model in female FoxN-1 mice. Animals (6 mice per group) were treated with three injections, with injections being given every fourth day (q7d schedule; on days 12, 16 and 20). Tumor growth was determined by direct caliper measurements. Mice were treated with saline, free cisplatin or a liposomal cisplatin. For both the free and liposomal-cisplatin treatments, the doses were 3.0 mg/kg cisplatin.

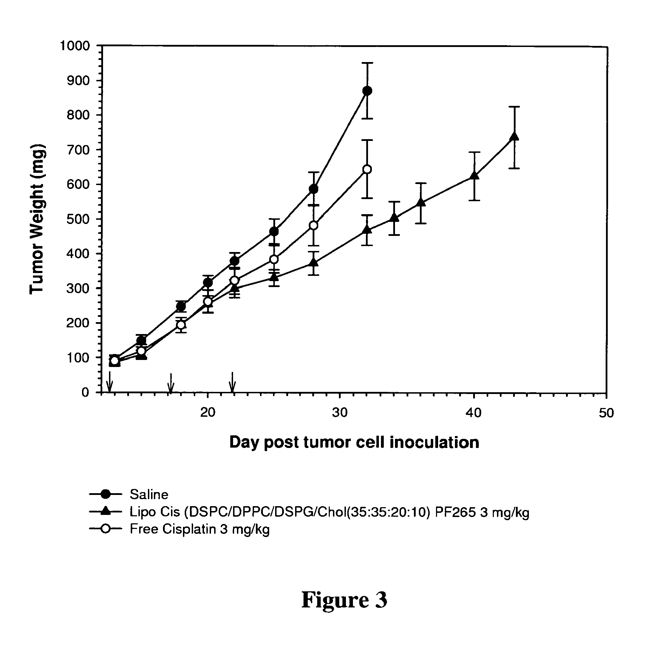

Results presented in Figure 3 (points represent mean tumor size +/- standard error of the mean (SEM) determined on the specified day) show that administration of cisplatin encapsulated in blended DSPC/DPPC/DSPG/Cholesterol liposomes resulted in a significant reduction in tumor burden compared to that of free cisplatin and saline controls.

Example 3 (Not within scope of present invention)

Combinations of Cisplatin and Other Therapeutic Agents Demonstrate Drug Ratio-Dependent Non-Antagonistic and Antagonistic Effects

Many combinations of two or more drugs have the ability to exhibit synergistic effects. Similarly, combinations of the same two or more drugs may show additive or antagonistic interactions depending upon the ratio, and often concentration, of drugs used. In order to identify ratios of platinum-based drugs and other therapeutic agents that are synergistic, combinations of cisplatin or carboplatin with various additional therapeutic agents were tested for their cytotoxic effects *in vitro.* More specifically, combinations that demonstrate synergy over a broad drug concentration range were identified.

Measuring additive, synergistic or antagonistic effects was performed using each drug combination at a number of mole ratios in human cancer cell lines. The standard tetrazolium-based colorimetric MTT cytotoxicity assay protocol (Mosmann, et al., J. Immunol Methods (1983) 65(1-2):55-63) was utilized to determine the readout for the fraction of cells affected. Briefly, viable cells reduce the tetrazolium salt, 3-(4,5-diethylthiazoyl-2-yl)-2,5-diphenyltetrazolium bromide (MTT) to a blue formazan which can be read spectrophotometrically. Cells, such as the human H460 non-small cell lung cancer cell line used here, are grown in 25 $cm^2$ flasks and passaged (passage number <20), resuspended in fresh cell culture medium and added into 96-well cell culture plates at a concentration of 1000 cells per well in 100 $\mu$L per well. The cells are then allowed to incubate for 24 hours at 37°C, 5% $CO_2$. Single-cell preparations such as these may also be prepared from patient tumors or biopsies by homogenizing the tissue with known techniques. The following day, serial drug dilutions are prepared in 12-well cell culture plates. The agents, previously prepared in various solutions, are diluted in fresh cell culture media. Agents are administered to the appropriate or specified wells for single agents (20 $\mu$L) and at specific fixed ratio dual agent combinations (increments of 20 $\mu$L) using a Latin square design or "checkerboard" dilution method. The total well volumes are made up to 200 $\mu$L with fresh media. The drug exposure is for a duration of 72 hours.

Following drug exposure, MTT reagent (1 mg/mL in RPMI) is added to each well at a volume of 50 $\mu$L per well and incubated for 3-4 hours. The well contents are then aspirated and 150 $\mu$L of dimethylsulfoxide (DMSO) is added to each well to disrupt the cells and to solubilize the formazan precipitate within the cells. The 96-well plates are shaken on a plate shaker, and read on a microplate spectrophotometer set at a wavelength of 570 nm. The optical density (OD) readings are recorded and the OD values of the blank wells (containing media alone) are subtracted from all the wells containing cells. The cell survival following exposure to agents is based as a percentage of the control wells (cells not exposed to drug). All wells are performed in triplicate and mean values are calculated.

A combination index was determined for each platinum drug/therapeutic agent dose using CalcuSyn which is based on Chou and Talalay's theory of dose-effect analysis, in which a "median-effect equation" has been used to calculate a number of biochemical equations that are extensively used in the art. Derivations of this equation have given rise to higher order equations such as those used to calculate Combination Index (CI). CI can be used to determine if combinations of more than one drug and various ratios of each combination are antagonistic (CI > 1.1), additive ($0.9 \leq$ CI $\geq 1.1$) or synergistic (CI < 0.9). CI plots are typically illustrated with CI representing the y-axis versus the proportion of cells affected, or fraction affected ($f_a$), on the x-axis. The data in Figures 4A-4D, plotted as CI versus the fraction of H460 cells affected, clearly illustrate that particular drug ratios are antagonistic while others are synergistic or additive.

Figure 4A shows that cisplatin and irinotecan at mole ratios of 8:1, 16:1, 32:1 and 64:1 were non-antagonistic over the vast majority of the range of $f_a$ values whereas a 4:1 ratio was synergistic at very low $f_a$ values yet strongly antagonistic above an $f_a$ value of 0.4. In contrast, the 1:1 and 2:1 ratios were antagonistic over a substantial range of $f_a$ values.

Figure 4B shows that cisplatin and edelfosine at mole ratios of 10:1 and 1:1 were also shown to exhibit distinct combination effects in H460 cells as summarized by the CI versus $f_a$ plot. The combination at a 10:1 mole ratio was non-antagonistic for approximately 50% of the fraction affected range at low concentrations and antagonistic at higher concentrations, while a 1:1 mole ratio demonstrated synergy over the entire concentration range.

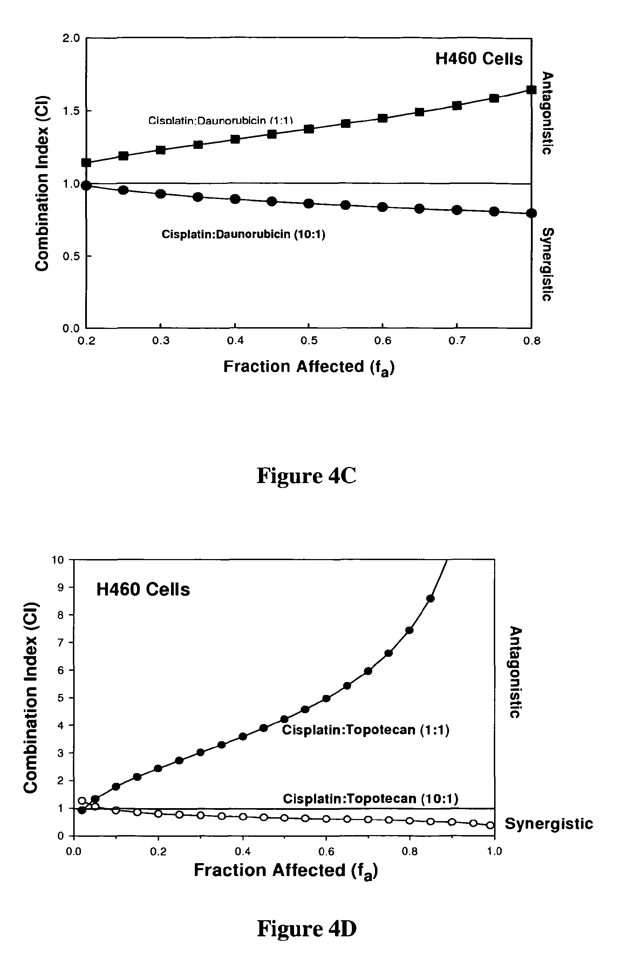

Figure 4C demonstrates that synergy was observed for a combination of cisplatin and daunorubicin at mole ratio of 10:1 over the entire $f_a$ range while the 1:1 mole ratios displayed antagonism over the complete $f_a$ range.

Cisplatin/topotecan combination data is presented in Figure 4D. A 10:1 mole ratio of this combination has a non-antagonistic interaction over a wide range of doses that affect 5% to 99% of cells ($f_a$ =0.05 to $f_a$=0.99). In contrast, cisplatin/topotecan at a 1:1 mole ratio was strongly antagonistic over the same $f_a$ range.

Example 4

Maintaining Synergistic Combinations Using Blended Liposomes

To determine if a synergistic ratio of a platinum-based drug and a second therapeutic agent could be maintained using blended liposomes *in vivo* studies were carried out on liposomal cisplatin and liposomal irinotecan formulated in separate blended DSPC/DPPC/DSPG/Cholesterol liposomes.

Cisplatin was loaded into DSPC/DPPC/DSPG/Cholesterol (35/35/20/10) liposomes containing 150 mM NaCl by incubating the drug with the liposomes for 60 minutes at 60°C in the presence of 7.5% ethanol at a cisplatin concentration of 5 mg/ml. The reaction was cooled to precipitate unencapsulated cisplatin or diluted with hot saline. The liposomes were then buffer exchanged into saline to remove unencapsulated cisplatin and subsequently into phosphate-buffered sucrose (pH 7).

Irinotecan was loaded into DSPC/DPPC/DSPG/Cholesterol (35/35/20/10) liposomes containing 300 mM TEA/10 mM sodium gluconate (pH 7) by first exchanging the external liposomal buffer for phosphate-buffered sucrose (pH 7), and then incubating the irinotecan with the liposomes at 41-43°C for 1 hour to achieve an irinotecan to lipid ratio of 0.17. The resulting irinotecan-containing liposomes were buffer exchanged into phosphate-buffered sucrose (pH 7). Analysis of cisplatin and irinotecan percent drug encapsulation was determined by atomic absorption spectrometry and HPLC analysis, respectively.

Liposomal irinotecan and liposomal cisplatin were co-administered to female CD-1 nude mice at the non-antagonistic mole ratio of 5:1 with doses of 1 mg/kg cisplatin, 10 mg/kg irinotecan and 130 mg/kg lipid. At the indicated time points (3 mice per time point), blood was collected by cardiac puncture and placed into EDTA coated microtainers. The samples were centrifuged and plasma was carefully transferred to another tube. Plasma irinotecan and cisplatin levels were determined by HPLC and atomic absorption spectrometry, respectively.

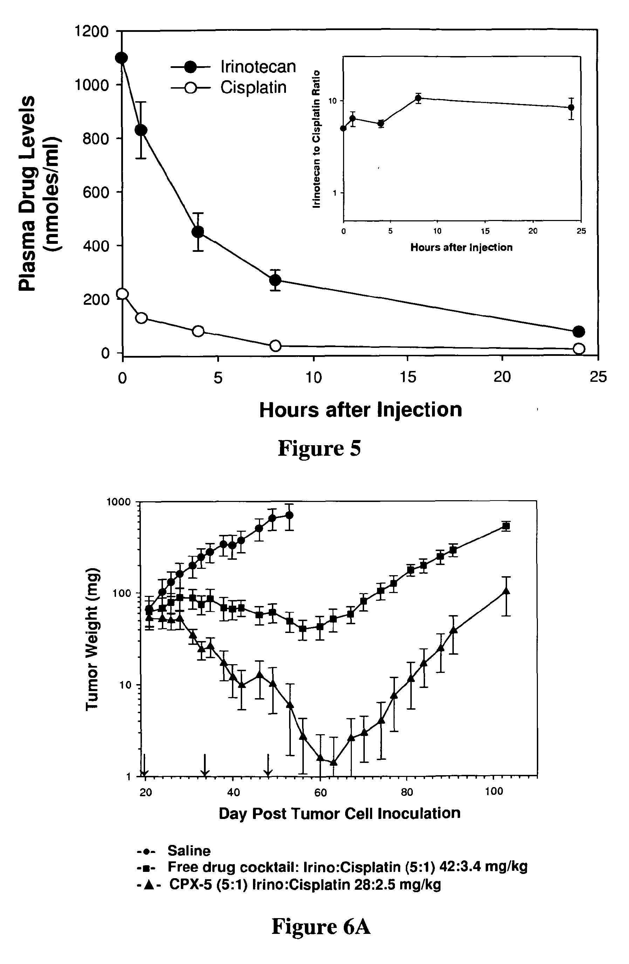

Results in Figure 5 (data points represent mean drug concentrations determined in plasma +/- SD at the specified time points) show that following intravenous injection of formulations containing cisplatin and irinotecan, loaded separately into blended DSPC/DPPC/DPPG/Cholesterol liposomes, the rates of plasma drug elimination were comparable and non-antagonistic drug ratios could be maintained over the 24-hour time course after administration as evidenced by the plasma concentrations of irinotecan (nmoles/mL) being roughly five times that of cisplatin (nmoles/mL) at various time points (see inset Figure 5).

Example 5

Blended Liposome-Encapsulated Drug Combinations demonstrate Enhanced Efficacy Compared to Free Drug Cocktails

Many therapies today, particularly combination chemotherapies, rely on administration of free drug cocktails. The investigators here wanted to establish whether enhanced efficacy is observed in blended liposome-encapsulated drug combinations compared to free drug cocktails of the same combinations. Efficacy studies were carried out using a combination of free cisplatin and irinotecan as well as co-administered liposomal cisplatin and liposomal irinotecan formulated separately in blended liposomes.

Cisplatin was loaded into DSPC/DPPC/DPPG/Cholesterol (35:35:20:10 mol %) liposomes and irinotecan was loaded into DSPC/DPPC/DPPG/Cholesterol (35:35:20:10 mol %) liposomes as described above.

Tumor studies on mice were performed as described in Example 1 except that mice were inoculated subcutaneously with either Capan-1 human pancreatic cancer cells or human H460 non-small cell lung cancer cells instead of P388 leukemia cells. Liposomal cisplatin and irinotecan combinations or free drug cocktails of the two drugs were administered to female_Foxn1 nude mice bearing the xenograft tumors. Mice were treated with a multiple dosing schedule (arrows indicate the days of treatment) of saline, free drug cocktail at an irinotecan:cisplatin 5:1 mole ratio, or a blended liposomal formulation of irinotecan:cisplatin co-administered at a 5:1 mole ratio ("CPX-551").

Figure 6A shows the results, where each data point represents mean tumor size +/-SEM determined on the specified day. The saline control (solid circles) did not inhibit tumor growth; similarly, the free cocktail (solid squares) showed only a slight effect on tumor growth. In comparison, the co-administered blended liposomal formulation, CPX-551 (solid triangles), showed significant tumor growth inhibition.

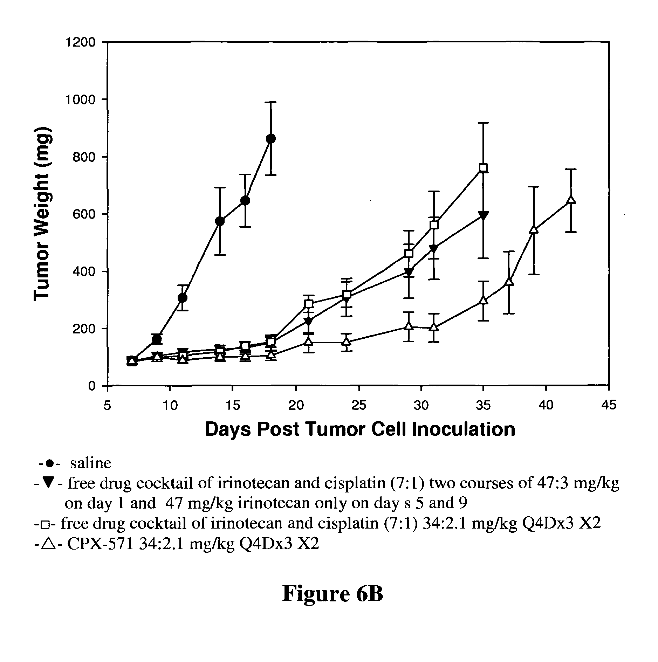

In order to fully compare the unencapsulated free drug cocktail to the liposomal co-formulation, we tested a variety of drug schedules for maximal therapeutic effect in the H460 NSCLC tumor model. The free drug schedules were compared with the liposomal co-formulation at a 7:1 ratio using Q7Dx3, Q4Dx3 and Q4Dx3X2 dosing schedules. In a separate experiment the unencapsulated drugs were tested using a dosing schedule mimicking the standard human clinical regimen (Chu and DeVita, 2006) in which both drugs are given on day 1 and then alternates with irinotecan independently for the following two doses per cycle. To achieve this type of schedule, the relative doses were modified to account for differences in potency between mice and humans. In a final experiment, the optimal schedule from the first two experiments was used to directly compare with a blended liposomal formulation of irinotecan:cisplatin co-administered at a 7:1 mole ratio ("CPX-571"). In this comparison, the human clinical regimen was inferior to simultaneous doses of both free drugs. Using the optimized dosing schedules, the CPX-571 liposomal co-formulation was superior to the unencapsulated drugs (Figure 6B). The log cell kill (LCK) values for the treatments were 2.29 and 2.20 for the most effective free drug therapies using either co-administration or alternating administration schedules respectively, and an increased LCK value of 3.34 was obtained for CPX-571, a value also considered highly active for antitumor agents (Cobertt, *et al.,* 2002).

Example 6

Combinations of Blended Liposome-Encapsulated Drugs Demonstrate Enhanced Efficacy Over Individual Agents Administered in the Same Blended Liposomes

The investigators here wanted to establish whether enhanced efficacy is observed in blended liposome-encapsulated drug combinations compared to each liposome-encapsulated drug administered alone. The therapeutic effect of liposome encapsulated cisplatin and irinotecan each administered separately was compared to both encapsulated drugs administered together at two different non-antagonistic ratios.

Cisplatin was loaded into DSPC/DPPC/DPPG/Cholesterol (35:35:20:10 mol %) liposomes and irinotecan was loaded into DSPC/DPPC/DPPG/Cholesterol (35:35:20:10 mol %) liposomes as described above.

Tumor-bearing Foxn1 mice were treated with a multiple dosing schedule (arrows indicate the days of treatment) of saline, liposomal-cisplatin and liposomal-irinotecan administered individually and together in order to compare the efficacy of single drug-loaded blended liposomes ("liposomal cisplatin" and "liposomal irinotecan") with blended liposomal-cisplatin and liposomal-irinotecan co-administered at either an irinotecan:cisplatin ratio of 5:1 or 7:1 ("CPX-551" or "CPX-571," respectively). Data points represent mean tumor size +/- standard error of the mean (SEM).

Results presented in Figure 7A show that administration of irinotecan and cisplatin encapsulated in blended DSPC/DPPC/DPPG/Cholesterol (35:35:20:10 mol %) liposomes and co-administered at a 5:1 irinotecan:cisplatin mole ratio ("CPX-551"; solid triangles) provided significantly better therapeutic activity in H460 tumor-bearing mice when compared to animals injected with either liposomal cisplatin (open circles) alone, liposomal irinotecan (open squares) alone or saline (solid circles).

Figure 7B similarly shows that co-administration of liposomal-cisplatin and liposomal-irinotecan in blended liposomes at a 7:1 irinotecan:cisplatin mole ratio (solid triangles) provides enhanced therapeutic activity in H69 tumor-bearing mice compared to treatment with the same dose of either liposomal cisplatin (open circles) or liposomal irinotecan (open squares) alone.

To quantify the contribution of each of the liposomal agents relative to the liposomal co-formulation, log cell kill (LCK) values were generated from the 7:1 ratio data presented in Figure 7B (see Figure 7C). The liposomal cisplatin dose of 8.3 $\mu$moles/kg (2.5 mg/kg) resulted in modest tumor growth delay of 14 days, with an LCK value of 0.72, while the liposomal irinotecan at a dose of 57.6 $\mu$moles/kg (39 mg/kg) resulted in growth delay of 38 days and a LCK value of 1.95. However, the liposomal co-formulation containing dose-matched irinotecan and cisplatin (57.6 $\mu$moles/kg and

8.3 μmoles/kg) resulted in tumor growth delay of 78 days and an LCK value of 4.0, far more than the tumor growth delay (52 days) or LCK values (2.67) predicted based on additive activity of the two components. It is notable that the LCK value of 4.0 for CX-571 is well above the 2.8 LCK value considered "highly active" for an antitumor agent against a solid tumor model (Cobertt, *et al.,* 2002).

Example 7

Tumor-Delivery of Synergistic versus Antagonistic Drug Combinations in Blended Liposomes

In order to assess the biological effect of antagonism, we evaluated differences between synergistic and antagonistic ratios of the co-formulated liposomal drugs *in vivo.* Using the high cisplatin synergistic region in which toxicity is predominated by the cisplatin component, we were able to change the ratios between a synergistic and an antagonistic molar ratio by varying the irinotecan dose without changing the cisplatin dose or the relative toxicity.

Cisplatin was loaded into DSPC/DPPC/DPPG/Cholesterol (35:35:20:10 mol %) liposomes and irinotecan was loaded into DSPC/DPPC/DPPG/Cholesterol (35:35:20:10 mol %) liposomes as described above.

Using the H1299 NSCLC human tumor xenograft model in Foxn1 nude mice, tumor growth delay was assessed by administering liposomal cisplatin at 11 μmoles/kg (3.3 mg/kg) together with liposomal irinotecan at 2.2 μmoles/kg (1.5 mg/kg) of liposomal irinotecan to generate a 1:5 ratio of irinotecan:cisplatin. This 1:5 ratio resulted in a log cell kill of 1.06 despite the relatively small amount of irinotecan that was given. However, when the irinotecan dose was increased 10-fold (22 μmoles/kg; 15 mg/kg), while maintaining the same cisplatin dose (11 μmoles/kg; 3 mg/kg) and thus generating a molar ratio of 2:1, a decrease in the log cell kill value to 0.65 occurred, a result consistent with *in vivo* antagonism relative to the 1:5 drug ratio formulation. It should be noted that maintenance of drug ratios *in vivo* was constant for each of the formulated irinotecan:cisplatin ratios (data not shown).

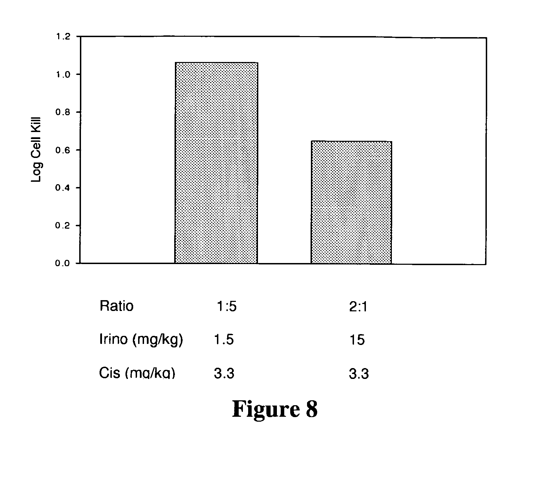

As seen in Figure 8 and stated above, irinotecan and cisplatin encapsulated in blended DSPC/DPPC/DPPG/Cholesterol (35:35:20:10 mol %) liposomes and co-administered at a 1:5 irinotecan:cisplatin mole ratio provided significantly better therapeutic activity when compared to animals injected with the same liposomes co-administered at a 2:1 ratio predicted to be antagonistic *in vitro.*

**Claims**

1. A composition for use in treating a hyperproliferative disease in a subject, which composition comprises:

   liposomes that consist of phosphatidylglycerol (DPPG) or distearoyl phosphatidylglycerol (DSPG); cholesterol (Chol); and equimolar amounts of distearoyl phosphatidylcholine (DSPC) and dipalmitoyl phosphatidylcholine (DPPC) encapsulating a platinum-based drug;
   in combination with a water-soluble camptothecin encapsulated in liposomes,
   wherein the liposomes encapsulating the platinum-based drug consist of DSPC:DPPC:DSPG:Chol or DSPC:DPPC:DPPG:Chol at the molar ratio 35:35:20:10.

2. The composition for use according to claim 1, wherein said platinum-based drug and said camptothecin are present in the composition in a mole ratio that has a non-antagonistic cytotoxic or cytostatic effect to relevant cells or tumor cell homogenates.

3. The composition for use according to claim 1 or 2, wherein the platinum-based drug is cisplatin, carboplatin or oxaliplatin.

4. The composition for use according to any of claims 1-3, wherein the camptothecin is irinotecan (CPT-11), topotecan, 9-aminocamptothecin or lurtotecan, or a hydrophilic salt thereof.

5. The composition for use according to any of claims 1-4, wherein the platinum-based drug and the camptothecin are encapsulated in the same liposomes.

6. The composition for use according to claim 1, wherein the liposomes consist of DSPC:DPPC:DSPG:Chol at the molar ratio 35:35:20:10 and contain irinotecan:cisplatin at a 5:1 or 7:1 ratio.

7. The composition for use according to claim 6, wherein the irinotecan and cisplatin are encapsulated in the same liposomes.

**Patentansprüche**

1. Zusammensetzung zur Verwendung in der Behandlung hyperproliferativer Erkrankungen bei einer Person, wobei die Zusammensetzung umfasst:

   Liposome, die aus Phosphatidylglycerol (DPPG) oder
   Distearoylphosphatidylglycerol (DSPG); Cholesterin (Chol); und äquimolaren Mengen von Distearoylphosphatidylcholin (DSPC) und
   Dipalmitoylphosphatidylcholin (DPPC) bestehen, die ein Platin-basiertes Pharmakon einkapseln;
   in Kombination mit einem wasserlöslichen Camptothecin eingekapselt in Liposome,
   wobei die Liposome, die das Platin-basierte Pharmakon einkapseln aus DSPC:DPPC:DSPG:Chol oder DSPC:DPPC:DPPG:Chol in einem molaren Verhältnis von 35:35:20:10 bestehen.

2. Zusammensetzung zur Verwendung nach Anspruch 1, wobei das Platin-basierte Pharmakon und das Camptothecin in der Zusammensetzung in einem Molverhältnis anwesend sind, das eine nicht-antagonistisch zytotoxische oder zytostatische Wirkung auf die relevanten Zellen oder Tumorzellhomogenate hat.

3. Zusammensetzung zur Verwendung nach Anspruch 1 oder 2, wobei das Platin-basierte Pharmakon Cisplatin, Carboplatin oder Oxaliplatin ist.

4. Zusammensetzung zur Verwendung nach einem der Ansprüche 1 bis 3, wobei das Camptothecin Irinotecan (CPT-11), Toptecan, 9-Aminocamptothecin oder Lurtotecan oder ein hydrophiles Salz davon ist.

5. Zusammensetzung zur Verwendung nach einem der Ansprüche 1 bis 4, wobei das Platin-basierte Pharmakon und das Camptothecin in die gleichen Liposome eingekapselt sind.

6. Zusammensetzung zur Verwendung nach Anspruch 1, wobei die Liposome aus DSPC:DPPC:DSPG:Chol in einem molaren Verhältnis von 35:35:20:10 bestehen und Irinotecan:Cisplatin in einem 5:1 oder 7:1 Verhältnis enthalten.

7. Zusammensetzung zur Verwendung nach Anspruch 6, wobei Irinotecan und Cisplatin in die gleichen Liposome eingekapselt sind.


**Revendications**

1. Une composition pour une utilisation dans le traitement d'une maladie hyper proliférative chez un sujet, laquelle composition comprend:

   - des liposomes consistant en phosphatidylglycérol (DPPG) ou distéaroyl phosphatidyl-glycérol (DSPG); cholestérol (Chol); et des quantités équimolaires de distéaroyl phosphatidylcholine (DSPC) et dipalmitoyl phosphatidylcholine (DPPC) encapsulant un médicament à base de platine;
   - en combinaison avec une camptothécine hydrosoluble encapsulée dans des liposomes,

   composition dans laquelle les liposomes encapsulant le médicament à base de platine consistent en DSPC/DPPC/DSPG/Chol ou DSPC/DPPC/DPPG/Chol selon un rapport molaire 35/35/20/10.

2. La composition pour une utilisation selon la revendication 1, dans laquelle le dit médicament à base de platine et ladite camptothécine sont présents en un rapport molaire dans la composition et qui présente un effet cytotoxique non-antagoniste ou un effet cytostatique vis-à-vis des cellules concernées ou des homogénats de cellules tumorales.

3. La composition pour une utilisation selon la revendication 1 ou 2, dans laquelle le médicament à base de platine est le cisplatine, le carboplatine ou l'oxaliplatine.

4. La composition pour une utilisation selon une quelconque des revendications 1 à 3, dans laquelle la camptothécine est l'irinotécan (CPT-11), le topotécan, la 9-aminocamptothécine ou le lurtotécan, ou un sel hydrophile en dérivant.

5. La composition pour une utilisation selon une quelconque des revendications 1 à 4, dans laquelle le médicament à base de platine et la camptothécine sont encapsulées dans les mêmes liposomes.

6. La composition pour une utilisation selon la revendication 1, dans laquelle les liposomes consistent en DSPC/DPPC/DSPG/Chol selon un rapport molaire 35/35/20/ 10 et contiennent irinotécan/cisplatine selon un rapport 5/1 ou 7/1.

7. La composition pour une utilisation selon la revendication 6, dans laquelle l'irinotécan et le cisplatine sont encapsulées dans les mêmes liposomes.

**Figure 1**

| Formulation | Tm (°C) | Thermogram | Cisplatin drug to lipid half life | MTD |
|---|---|---|---|---|
| DSPC/DSPG/Chol (70:20:10) | 54.9 | | 24 hours | 1 mg/kg |
| DSPC/DPPC/DSPG/Chol (35:35:20:10) | 48.8 | | 6.5 hours | ND |
| DSPC/DPPC/DPPG/Chol (35:35:20:10) | 45.5 | | 5 hours | >4 mg/kg |
| DSPC/DPPC/DPPG/Chol (17.5:52.5:20:10) | 43.9 | | 4 hours | ND |
| DPPC/DPPG/Chol (70:20:10) | 42.2 | | 4 hours | 4 mg/kg |

40  45  50  55

# Figure 2

**Figure 3**

**Irinotecan:Cisplatin Ratio in Human H460 Cells**

**Figure 4A**

**Figure 4B**

Figure 4C

Figure 4D

**Figure 5**

-•- Saline
-■- Free drug cocktail: Irino:Cisplatin (5:1) 42:3.4 mg/kg
-▲- CPX-5 (5:1) Irino:Cisplatin 28:2.5 mg/kg

**Figure 6A**

- ● - saline
- ▼ - free drug cocktail of irinotecan and cisplatin (7:1) two courses of 47:3 mg/kg
      on day 1 and 47 mg/kg irinotecan only on day s 5 and 9
- □ - free drug cocktail of irinotecan and cisplatin (7:1) 34:2.1 mg/kg Q4Dx3 X2
- △ - CPX-571 34:2.1 mg/kg Q4Dx3 X2

**Figure 6B**

**Figure 7A**

(●) saline
(▲) 2.5 mg/kg liposomal cisplatin
(■) 39 mg/kg liposomal irinotecan
(▼) CPX-571 containing 39 and 2.5 mg/kg irinotecan and cisplatin

**Figure 7B**

| Treatment | Dose (mg/kg) | Growth Delay (Days) | % Growth Delay %(T-C)/C[A] | Log$_{10}$ cell kill[B] (LCK) |
|---|---|---|---|---|
| Lipo-Irinotecan | 39 | 38 | 149% | 1.95 |
| Lipo-Cisplatin | 2.5 | 14 | 55% | 0.72 |
| CPX-571 | 39:2.5 | 78 | 304% | 4.0 |

[A] Based on tumors to reach 400 mg.
[B] Formula (T-C)/ (3.32)(Td)  Td – tumor doubling time

**Figure 7C**

| Ratio | 1:5 | 2:1 |
|---|---|---|
| Irino (mg/kg) | 1.5 | 15 |
| Cis (mg/kg) | 3.3 | 3.3 |

**Figure 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5945122 A
- US 6126966 A
- US 6511676 B
- WO 04087105 A
- EP 0280492 A2
- EP 0225130 A2
- WO 2007035783 A2
- US 6545010 B
- US 20030147945 A
- US 5616341 A
- US 5736155 A
- US 5785987 A
- US 5837282 A
- US 7238367 B
- WO 07076117 A
- US 3993754 A, Rahman
- US 4145410 A, Sears
- US 4235871 A, Papahadjopoulos
- US 4224179 A, Schneider
- US 4522803 A, Lenk
- US 4588578 A, Fountain

### Non-patent literature cited in the description

- **THATCHER et al.** *Ann Oncol,* 1995, vol. 6, S83-S95
- **MEERUM et al.** *Cancer Chemother Pharmacol,* 2002, vol. 49, 21-210
- **JEHN et al.** *Anticancer Res.,* January 2007, vol. 27 (1A), 471-5
- **HWANG et al.** *J. of Dermatological Science,* April 2007, vol. 46 (1), 11-20
- **TAKADA et al.** *Proc. Amer. Assoc. Cancer Res.,* 1992, vol. 33, 226
- **KANO et al.** *Int. J. Cancer,* 1992, vol. 50, 604
- **KUDOH et al.** *Jpn. J. Cancer Res.,* 1993, vol. 84, 203
- **ZASTRE et al.** *Cancer Chemother. Pharmacol.,* 2006
- **BANGHAM et al.** *J. Mol. Biol.,* 1965, vol. 12, 238
- **DEAMER ; BANGHAM.** *Biochim. Biophys. Acta,* 1976, vol. 443, 629
- **SZOKA ; PAPAHADJOPOULOS.** *P.N.A.S.,* 1978, vol. 75, 4194
- **LOEWE et al.** *Arzneim-Forsch,* 1953, vol. 3, 285-290
- **STEEL et al.** *Int. J. Radiol. Oncol. Biol. Phys.,* 1979, vol. 5, 27-55
- **WEBB.** Enzyme and Metabolic Inhibitors. Academic Press, 1963, vol. 1, 1-5
- **CHOU.** *J. Theor. Biol.,* 1976, vol. 39, 253-76
- **CHOU.** *Mol. Pharmacol.,* 1974, vol. 10, 235-247
- **ZOLI et al.** *Int. J. Cancer,* 1999, vol. 80, 413-416
- **PANNACCIULLI et al.** *Anticancer Res.,* 1999, vol. 19, 409-412
- **BERENBAUM.** *Pharmacol. Rev.,* 1989, vol. 41, 93-141
- **GRECO et al.** *Pharmacol Rev.,* 1995, vol. 47, 331-385
- **CHOU ; TALALAY.** *Adv. Enzyme Reg.,* 1984, vol. 22, 27-55
- **CHOU et al.** Synergism and Antagonism in Chemotherapy. Academic Press, 1991, 223-244
- Dose-effect analysis with microcomputers: quantitation of ED50, LD50, synergism, antagonism, low-dose risk, receptor ligand binding and enzyme kinetics. **CHOU ; CHOU.** CalcuSyn Manual and Software. 1987
- CalcuSyn Manual and Software; Cambridge: Biosoft. 1987
- **MOSMANN.** *J. Immunol. Methods,* 1983, vol. 65 (1-2), 55-63
- **MOSMANN et al.** *. Immunol Methods,* 1983, vol. 65 (1-2), 55-63